Europäisches Patentamt

(19) European Patent Office

Office européen des brevets

(11) Publication number: **0 384 600 B1**

(12) **EUROPEAN PATENT SPECIFICATION**

(45) Date of publication of patent specification :
04.08.93 Bulletin 93/31

(51) Int. Cl.⁵ : **A61K 31/60, A61K 9/20**

(21) Application number : **90301151.8**

(22) Date of filing : **05.02.90**

(54) **Salsalate tablet.**

(30) Priority : **24.02.89 US 314788**

(43) Date of publication of application :
**29.08.90 Bulletin 90/35**

(45) Publication of the grant of the patent :
**04.08.93 Bulletin 93/31**

(84) Designated Contracting States :
**DE GB IT**

(56) References cited :
**EP-A- 0 174 006
GB-A- 2 058 565
US-A- 4 555 399
Physicians Desk Reference, 1988, 42, p. 1678**

(73) Proprietor : **RIKER LABORATORIES, INC.
P.O. Box 33427
Saint Paul Minnesota 55133 (US)**

(72) Inventor : **Beaurline, Joseph M.
c/o Riker Laboratories Inc., 3M Center
St. Paul, Minnesota 55144-1000 (US)**
Inventor : **Schultz, Robert K.
c/o Riker Laboratories Inc., 3M Center
St. Paul, Minnesota 55144-1000 (US)**

(74) Representative : **Baillie, Iain Cameron et al
Ladas & Parry Altheimer Eck 2
W-8000 München 2 (DE)**

EP 0 384 600 B1

## Description

This invention pertains to pharmaceutical formulations. More particularly this invention pertains to tablets containing salsalate. In another aspect, this invention pertains to the masking of taste and esophageal irritation of pharmaceutical formulations, particularly tablets.

Salsalate (salicylsalicylic acid; 2-hydroxybenzoic acid 2-carboxyphenyl ester) is a nonsteroidal anti-inflammatory drug (NSAID). Salsalate has a very unpleasant taste and causes irritation of the mucous membranes of the esophagus. Known salsalate tablets overcome this problem by either film coating or by including excipients in an amount great enough to mask the taste and irritation. For example, DISALCID™ (commercially available from Riker Laboratories, Inc., St. Paul, MN) is supplied as a tablet coated with hydroxypropyl methylcellulose and additionally containing magnesium stearate, microcrystalline cellulose, polyethylene glycol, polysorbate 80, starch, talc, and dye. Physicians Desk Reference, 1988, 42, 1678.

Salsalate is generally non-compressible and shows a wide variety of tableting characteristics depending on the method of manufacture. Salsalate tablets can be difficult to compress and may be subject to internal lamination, which may lead to a catastrophic tablet failure known as capping. Capping and its relation to axial and radial tensile strength are discussed in Acta Pharm. Suecica, 1984, 21, 1. Hydroxypropyl cellulose is a known pharmaceutic aid. It has been used as a binder, a film former, and as a granulating agent in both conventional and sustained release formulations. The use of hydroxypropyl cellulose in salsalate formulations is known. For example, U.S. Pat. No. 4,666,716 discloses an anti-diarrheal composition containing an NSAID such as salsalate and a polymeric hydroabsorptive agent in a ratio of between about 1:30 and about 1:600. The composition can be in the form of a tablet containing a binder such as hydroxypropyl cellulose. It is notable that these compositions contain the NSAID as a relatively minor component (i.e., at most several percent of the total weight of the composition).

U.S. Pat. No. 4,789,667 discloses pharmaceutical compositions comprising an effective amount of drug and an effective amount of a pyroglutamate as a skin-penetration enhancer. A variety of drugs, presentations and excipients are disclosed. The composition can be in the form of a tablet, the drug can be an anti-inflammatory agent such as salsalate, and excipients in a tablet can include hydroxypropyl cellulose. The amounts of drug and excipient in a tablet are not addressed.

The present invention provides novel tablets containing salsalate, microcrystalline cellulose, hydroxypropyl cellulose as a binder substantially uniformly dispersed in the tablet, and a lubricant. Additional components incorporated in preferred embodiments include a conventional disintegrant such as a starch or a pre-gelatinized starch, or a disintegrant that is effective when used in a lesser quantity than that required when a conventional disintegrant is used. The latter class of disintegrants are referred to herein as superdisintegrants. A tablet of the invention exhibits at least 85% dissolution when tested according to the dissolution assay set forth below.

One presently preferred embodiment of the invention provides a tablet comprising from 80 to 90 percent by weight of salsalate, 5 to 15 percent by weight of microcrystalline cellulose, 1.5 to 5.5 percent by weight of hydroxyprooyl cellulose, 0.15 to 0.5 percent by weight of a lubricant, and 0.2 to 1.5 percent by weight of a superdisintegrant, all weights being based on the total weight of the tablet.

A second embodiment of the present invention provides a tablet comprising from 81 to 89 percent by weight of salsalate, 5 to 12 percent by weight of microcrystalline cellulose, 1.1 to 2.5 percent by weight of hydroxypropyl cellulose, 0.15 to 0.4 percent by weight of a lubricant, and 3.5 to 8 percent by weight of corn starch, all weights being based on the total weight of the tablet. Preferably this tablet comprises salsalate present in an amount of 84.4 percent by weight based on the total weight of the tablet; microcrystalline cellulose present in an amount of 5.6 percent by weight based on the total weight of the tablet; hydroxypropyl cellulose LF present in an amount of 2 percent by weight based on the total weight of the tablet; magnesium stearate present in an amount of 0.2 percent based on the total weight of the tablet; and corn starch present in an amount of 7.8 percent by weight based on the total weight of the tablet.

A third embodiment of the present invention provides a tablet comprising from 81 to 85 percent by weight of salsalate, 13 to 18 percent by weight of microcrystalline cellulose, 1 to 5 percent by weight of hydroxypropyl cellulose, and 0.1 to 0.5 percent of a lubricant, all weights being based on the total weight of the tablet.

The inclusion of hydroxypropyl cellulose in the tablets of the invention eliminates the need for a discrete outer film coating, thus reducing production costs. Furthermore, the quantity of excipient is reduced, allowing a smaller tablet for a given dose relative to known tablets. The inclusion of hydroxypropyl cellulose also allows the salsalate to be formulated into tablets that have superior mechanical strength independent of the method of salsalate manufacture.

The salsalate component of the present invention is preferably a powder. Preferably no more than 1 percent of the salsalate particles will be retained by a sieve with 0.841 mm sieve openings (a No.20 sieve).

The microcrystalline cellulose component of the present invention serves as a combination binder and disintegrant. Microcrystalline cellulose is described as purified, partially depolymerized cellulose prepared by treating alpha cellulose, obtained as a pulp from fibrous plant material, with mineral acids. National Formulary XV, p. 1218. A preferred microcrystalline cellulose is Avicel™ PH101 (available from the FMC Corporation).

The hydroxypropyl cellulose component is a binder substantially uniformly dispersed in the tablet. It is thought that the hydroxypropyl cellulose becomes plastic under the pressures exerted by a conventional tableting apparatus. Upon pressing, the hydroxypropyl cellulose flows to surround the salsalate. The resulting tablet, without a discrete outer coating, effectively masks the taste and esophageal irritation of the salsalate. The hydroxypropyl cellulose preferably has a molecular weight of between 75,000 and 125,000. A preferred hydroxypropyl cellulose is Klucel™ LF (available from Hercules, Inc.). .

The lubricant component of the present invention may be any pharmaceutically acceptable lubricant. Such lubricants include silicone fluids, hydrogenated vegetable oils, microfine silica, stearate salts, stearic acid, polyethylene glycol, talc, sodium benzoate, sodium acetate, magnesium carbonate, and magnesium oxide. The lubricant is present in an amount sufficient to provide acceptable die release properties to the tablets. The preferred lubricant is magnesium stearate.

The superdisintegrant component of a preferred tablet of the present invention is a disintegrant that is effective when used in a substantially lesser quantity than that required when a conventional disintegrant is used, and is selected from crosslinked polyvinylpyrrolidone, sodium starch glycolate, and crosslinked carboxymethyl cellulose. The preferred superdisintegrant is a crosslinked sodium carboxymethyl cellulose known as croscarmellose sodium (Type A) (available from FMC Corporation as Ac-Di-Sol™).

The conventional disintegrant component is a common starch (e.g., corn starch) or a pregelatinized starch. Such materials can be obtained from any starch manufacturer such as, for example, the National Starch Corporation.

A preferred table! of the present invention comprises from 80 to 90 percent, preferably 84 percent, by weight of salsalate; from 5 to 15 percent, preferably 11.1 percent, by weight of microcrystalline cellulose; 1.5 to 5.5 percent, preferably 4 percent, by weight of hydroxypropyl cellulose; 0.15 to 0.5 percent, preferably 0.45 percent, by weight of a lubricant; and 0.2 to 1.5 percent, preferably 0.7 percent, by weight of a superdisintegrant, all weights being based on the total weight of the tablet.

Such tablets are prepared by granulating a preblended mixture of salsalate, microcrystalline cellulose and superdisintegrant using a solution of 10 to 19 percent, preferably 12 percent, hydroxypropyl cellulose in water as the granulating liquid. Alternatively, dry hydroxypropyl cellulose can be included in the preblend and water used as the granulating liquid. The granulation is dried until the loss-on-drying is less than 1%, then sized by oscillation through a 12 to 14 mesh screen. A "12 mesh" "screen" represents a 1.68 mm sieve opening and a 0.810 mm wire diameter; and a "14 mesh" "screen" represents a 1.41 mm sieve opening and a 0.725 mm wire diameter. A portion, not more than about one-fourth, of the dried and sized granulation is mixed with the lubricant and this premix is then mixed with the remainder of the granulation. The lubricated granulation is compressed into tablets using conventional tablet presses.

Another tablet of the present invention comprises from 81 to 89 percent, preferably 84 percent, by weight of salsalate; 5 to 12 percent, preferably 6 percent, by weight of microcrystalline cellulose; 1.1 to 2.5 percent, preferably 2 percent, by weight of hydroxypropyl cellulose, and 0.15 to 0.4 percent, preferably 0.2 percent, by weight of a lubricant, and 3.5 to 8 percent, preferably 8 percent, by weight of corn starch; all weights being based on the total weight of the tablet.

Tablets of the above embodiment are prepared by granulating a preblended mixture of salsalate and corn starch using a solution of 12 to 19 percent hydroxypropyl cellulose in water as the granulating liquid. The granulation is dried until the loss-on-drying is less than 1 %, then sized by oscillation through a 1.68 mm sieve opening (a 12 mesh screen). The dried, sized granulation is blended with the microcrystalline cellulose. A portion of the blend, not more than one-fourth, is mixed with the lubricant. This premix is blended with the remainder of the granulation. The lubricated granulation is compressed into tablets using conventional tablet presses.

Another tablet of the invention comprises from 81 to 85 percent by weight of salsalate, 13 to 18 percent by weight of microcrystalline cellulose, 1 to 5 percent by weight of hydroxypropyl cellulose, and 0.1 to 0.5 percent by weight of a lubricant, all weights being based on the total weight of the tablet. These tablets are made by granulating a preblended mixture of salsalate and microcrystalline cellulose using a solution of 4 to 6 percent hydroxypropyl cellulose in water as the granulating liquid. The granulation is dried and sized as described above. A portion is mixed with a lubricant and the resulting premix is put through a 1.68 mm sieve opening (a 12 mesh screen) then mixed with the remainder of the granulation. The lubricated granulation is compressed into tablets using conventional tablet presses.

A tablet of the invention exhibits disintegration and dissolution properties consistent with conventional, not sustained, release of salsalate. A tablet of the invention exhibits at least 85% dissolution when tested according to the dissolution assay set forth below (hereinafter referred to as the "Dissolution Assay"). For the purposes of the instant specification and claims, such dissolution values are the average of 6 independent determinations unless otherwise noted.

Preferably, tablets possess sufficient strength to withstand mechanical handling. One method of characterizing the mechanical strength of a tablet is by measuring the tensile strength. The ratio of the axial tensile strength to the radial tensile strength gives an isotropy ratio (G. Alderborn and C. Nystrom, Acta Pharmaceutic Suecica, 21, 1-8 (1984) or index of capping (P. Jarosz and E. Parrott, Journal of Pharmaceutical Sciences, 71, 607-614 (1982). The higher the value of the isotroPy ratio the lesser the probability of capping. It is preferred that a tablet of the invention that has been compressed at a compression force of 1090 kg exhibit a ratio of axial/radial tensile strength of at least 0.25, more preferably at least 0.3, and most preferably at least 0.4. Likewise, the higher the axial tensile strength, the lesser the probability of capping. It is preferred that a tablet of the invention that has been compressed at a compression force of 1500 kg exhibit an axial tensile strength of at least 3.0 kg/cm$^2$, more preferably at least 4.0 kg/cm$^2$, and most preferably at least 5.0 kg/cm$^2$. For the purposes of the instant specification and claims, all such values are the average values obtained for 10 tablets according to the test methods set forth below.

The following examples and tables are provided to illustrate the invention. The hydroxypropyl cellulose used in the examples was Klucel™ LF from Hercules, Inc. As used herein, friability means the friability measured on a Roche Friabilator for 20 tablets and 100 revolutions, and hardness means the average tablet hardness of ten tablets as measured on a Schleuniger Hardness Tester. Disintegration time, dissolution, axial tensile strength, and radial tensile strength are determined using the test methods described below.

## Disintegration Time

The disintegration time is determined using a disintegration apparatus (USP XX) and USP gastric fluid without enzymes. 800 ml of simulated gastric fluid is placed in a 1 liter beaker. The beaker is placed in a constant temperature bath and the temperature of the fluid is allowed to reach 37±2 °C. One tablet is placed in each of the 6 tubes of a basket. A disk is placed in each tube. The disk is oriented so that the flat surface with the greater area faces downward. The basket is positioned in the disintegration apparatus and the actuator is immediately started. The apparatus is operated at 37±2 °C. The exact total elapsed time required for each tablet to disintegrate and completely pass through the screen is recorded. The disintegration time is the average of the time recorded for the 6 tablets.

## Dissolution Assay

A dissolution apparatus conforming to the requirements for USP Apparatus 2 is used. 900 ml of 0.25 M phosphate buffer solution having a pH of 7.4 is placed in a dissolution vessel. The paddle speed is set at 50 rpm and the dissolution medium is allowed to equilibrate to 37 °C. One tablet is placed in the vessel. After 45 minutes a sample is withdrawn and analyzed for salicylsalicylic acid content using a UV spectrophotometer set at 308 nm. The amount of dissolved salicylsalicylic acid is reported as a percent of the claimed tablet salicylsalicylic acid content.

## Axial Tensile Strength

The axial tensile strength of a tablet is measured as follows. A tablet is fastened between the heads of two 12.7 mm hex bolts using a thin coating of a quick set epoxy cement. The epoxied tablet is allowed to cure for 24 hours. The axial strength is measured by pulling the tablets apart using an Instron Model 1122 equipped with a set of pneumatics jaws (crosshead speed at 5.0 mm/min). Only data from tablets that break in the body of the tablet are used. If the tablet breaks at the epoxy-bolt interface the data are rejected. The calculation of $\sigma_A$ in kg/cm$^2$ from the Instron data is done using the following equation

$$\sigma_A = \frac{4F}{\pi D^2}$$

where F is the force to pull the tablet apart in kg, and D is the diameter of the tablet in cm. The value reported is the average of the values obtained for ten tablets.

## Radial Tensile Strength

The radial tensile strength of a tablet is measured using an Instron Model 1122 with a crosshead speed of 5.0 mm/min. Only data from tablets that exhibit a tensile break are used. Calculation of the radial tensile

EP 0 384 600 B1

strength $\sigma_R$ in kg/cm$^2$ is done using the equation below

$$\sigma_R = \frac{2F_c}{\pi Dt}$$

where $F_c$ is the force to crush the tablet in kg, D is the diameter of the tablet in cm, and t is the thickness of the tablet in cm. The value reported is the average of the values obtained for ten tablets.

Example 1

Using a 18.4 l Hobart mixer, 3.750 of salicylsalicylic acid (SSA) powder was blended with 170 g of corn starch for 5 minutes at low speed (#1). 60.0 g of hydroxypropyl cellulose was dissolved in 294.4 g of water and the resulting solution was added to the SSA/corn starch blend with mixing. The mixture was granulated for 5 minutes then dried in a Glatt fluid bed drier for 45 min at 60°C. The granulation was then oscillated through a 1.68 mm sieve opening (a 12 mesh screen). The resulting granulation has a bulk density of 0.671 g /ml. Using a 11.1 l Hobart mixer, 1,592 g of the granulation was blended with 100 g of Avicel™ PH101 at low speed for 5 minutes. Approximately one-fourth of this blend was placed in a plastic bag and mixed with 3.0 g of magnesium stearate (dense). The resulting premix was added to the remainder of the granulation in the Hobart mixer and mixed for 1 minute at speed #1. The granulation was compressed on a Stokes BB-2 press equipped with 12.7 mm flat faced beveled edged tooling and a tapered die using a compressive force of approximately 1320 kg. The composition of the resulting tablets is shown in Table 1. The tablets were found to be nonirritating to the esophagus. The axial and radial tensile strengths were measured according to the methods described above.

Table 1

| Component | mg/tablet |
|---|---|
| Salicylsalicylic acid | 750.0 |
| Corn Starch | 34.0 |
| Hydroxypropyl cellulose | 12.0 |
| Microcrystalline cellulose | 50.0 |
| Magnesium stearate (dense) | 1.5 |

Tensile Strength

| | |
|---|---|
| Axial tensile strength | $6.6 \pm 1.3$ kg/cm$^2$ |
| Radial tensile strength | $8.9 \pm 0.9$ kg/cm$^2$ |
| Axial/radial | 0.74 |

Example 2

Using the general method of Example 1, tablets having the composition and tensile strengths shown in Table 2 were prepared. The hydroxypropyl cellulose was added as a 13.9% solution in water. The bulk density of the initial (before the addition of the microcrystalline cellulose and magnesium stearate) granulation was 0.618 g/cc. The tablets did not irritate the esophagus.

Example 3

Using the general method of Example 1, tablets having the composition and tensile strengths shown in Table 2 were prepared. The hydroxypropyl cellulose was added as a 14.1 % solution in water. The bulk density of the initial granulation was 0.654 g/ml. The tablets were found to be nonirritating to the esophagus.

5

Example 4

Using the general method of Example 1, tablets with the composition and tensile strengths shown in Table 2 were prepared. The hydroxypropyl cellulose was added as a 18.1 % solution in water. The bulk density of the initial granulation was 0.618 g/ml. The tablets did not irritate the esophagus.

Example 5

Using the general method of Example 1, tablets having the composition and tensile strengths shown in Table 2 were prepared. The hydroxypropyl cellulose was added as a 15.7% solution in water. The bulk density of the initial granulation was 0.660 g/cc. The tablets die not irritate the esophagus.

## Table 2

### Example Number

| Component | 2 | 3 | 4 | 5 |
|---|---|---|---|---|
| | | mg/tablet | | |
| SSA | 750.0 | 750.0 | 750.0 | 750.0 |
| Corn starch | 69.0 | 46.0 | 51.0 | 50.0 |
| HPC | 18.0 | 12.0 | 18.0 | 15.0 |
| Avicel™ PH101 | 50.0 | 50.0 | 50.0 | 50.0 |
| Mg Stearate | 1.5 | 1.5 | 1.5 | 1.5 |

| Tensile Strengths | kg/cm$^2$ | | | |
|---|---|---|---|---|
| Axial | 8.8±1.1 | 8.0±1.0 | 9.5±1.7 | 8.2±1.2 |
| Radial | 11.1±0.7 | 9.9±0.6 | 11.1±0.7 | 10.6±0.6 |
| Axial/Radial | 0.79 | 0.81 | 0.86 | 0.77 |

SSA = Salicylsalicylic acid; HPC = hydroxypropyl cellulose

Example 6

Using a 11.1 l Hobart mixer, 3,750 g of salicylsalicylic acid powder was blended with 250 g of corn starch at low speed for 5 minutes. A solution of 75.0 g of hydroxypropyl cellulose (LF) in 550 g of water was added while blending. The granulation time was 9.5 minutes. The granulation was dried in a fluid bed drier at 60 °C for 60 minutes then oscillated through a 1.68 mm sieve opening (a 12 mesh screen). The resulting granulation had a bulk density of 0.535 g/cc. Using a 11.1L Hobart mixer, 1728 g of the granulation was blended with 212.0 g of Avicel™ PH101 at low speed for 5 minutes. A portion (not more than one fourth) of the resulting granulation was placed in a plastic bag and mixed with 3.2 g of magnesium stearate (dense). The resulting premix was added to the remainder of the granulation in the mixer and blended at low speed for 1 minute. A portion of the granulation was compressed on a Stokes BB-2 press equipped with 12.7 mm flat faced beveled edged tooling and a tapered die using three different compressive forces. These tablets were used to measure axial and radial tensile strengths (Table 4). The remainder of the granulation was compressed on a Stokes BB-2 press equipped with 7.9 mm by 19.0 mm standard cup upper bisect tooling and a tapered die. The composition of the tablets is shown in Table 3. These tablets were used to measure hardness, thickness, friability and dissolution (Table 4). Both types of tablets were found to be nonirritating to the esophagus.

Table 3

| Component | mg/tablet |
|---|---|
| Salicylsalicylic acid | 750.0 |
| Corn starch | 50.0 |
| Hydroxypropyl cellulose LF | 15.0 |
| Avicel™ PH101 | 100.0 |
| Magnesium stearate (dense) | 1.5 |

Table 4

| Compressive Force (kg) | 760 | 1090 | 1410 |
|---|---|---|---|
| Axial TS (kg/cm$^2$) | 20.0 | 18.2 | 23.8 |
| Radial TS (kg/cm$^2$) | 20.8 | 32.2 | 37.4 |
| Axial/Radial | 0.96 | 0.57 | 0.64 |
| Hardness (kp) | 10.6$\pm$1.5 | 15.4$\pm$2.2 | 19.1$\pm$1.3 |
| Thickness (mm) | 7.03$\pm$0.01 | 6.78$\pm$0.02 | 6.62$\pm$0.01 |
| Friability | 0.18 % | 0.09 % | 0.01 % |
| Dissolution | 96.9 % | 99.2 % | 98.4 % |
| (average of 3 tablets) | | | |

Example 7

Using a 11.1 l Hobart mixer, 750.0 g of salicylsalicylic acid powder was blended with 150.0 g of Avicel™ PH101 for 5 minutes at low speed. A solution of 19.0 g of Klucel™ LF in 380 g of water was added to the blended powders. The granulation time was 1 minute. The granulation was dried in a fluid bed drier for 45 minutes at 60 °C then oscillated through a 1.68 mm sieve opening (a 12 mesh screen). Approximately 200g of the granulation was combined with 1.35g of magnesium stearate (dense) in a glass jar, blended on a Turbula mixer at medium high speed for 30 seconds and then put through a 1.68 mm sieve opening (a 12 mesh screen). The resulting premix was blended with the remainder of the granulation in a Hobart mixer for 1 minute at low speed. A portion of the granulation was compressed on the Stokes BB-2 press equipped with 12.7 mm flat faced beveled edged tooling and a tapered die using a compressive force of about 1680 kg. These tablets were used to measure tensile strengths (Table 5). The remainder of the granulation was compressed on a Stokes BB-2 press equipped with 19.0 mm by 7.9 mm standard cup upper bisect tooling and a tapered die using a compressive force of either 1350 kg or 1675 kg. These tablets were used to determine hardness, thickness, and friability (Table 5). Both types of tablets were found to be nonirritating to the esophagus.

Table 5

| Component | mg/tablet | |
|---|---|---|
| Salicylsalicylic acid | 750.0 | |
| Avicel™ PH101 | 150.0 | |
| Klucel™ LF | 19.0 | |
| Magnesium stearate (dense) | 1.5 | |
| Tensile strengths | $kg/cm^2$ | |
| Axial | $13.48 \pm 3.47$ | |
| Radial | $20.55 \pm 0.68$ | |
| Axial/Radial | 0.66 | |
| | | |
| Compressive Force (kg) | 1350 | 1675 |
| Hardness (kp) | $16.1 \pm 1.4$ | $19.1 \pm 0.6$ |
| Thickness (mm) | $6.48 \pm 0.02$ | $6.35 \pm 0.02$ |
| Friability | 0.05 % | 0.07% |

Example 8

A hydroxypropyl cellulose solution was prepared by slowly adding 480 g of hydroxypropyl cellulose LF to 4892 ml of water with stirring. The stirring was continued until dissolution was complete. 5000 g of salicylsalicylic acid powder, 665 g of Avicel™ PH101 and 40 g of Ac-Di-Sol™ were placed in a Fuji vertical granulator equipped with a 35° triple blade and mixed for 5 minutes with the blade speed set at 300 rpm and the cross-screw set at low speed. The hydroxypropyl cellulose solution was added to the powder mass at a rate of 110 ml/min. The blade speed was set at 450 rpm and the cross-screw was set at high speed. After 17 minutes the powder mass still looked dry. The granulation process was continued with the blade speed reduced to 400 rpm then to 300 rpm. After 5 minutes, the mass appeared to be uniformly wetted. It was beaten for 1 minute with the blade speed at 150 rpm. The granulation was dried in a fluid bed drier at 60°C until the loss-on-drying was less than 1%. The bulk density was 0.576 g/ml. 2378 g of the granulation was placed in a 11.1 l Hobart mixer and blended with 4.0 g of magnesium stearate (dense) for 4 minutes at low speed (#1). The granulation was compressed on a Stokes BB-2 press equipped with 12.7 mm flat faced beveled edged tooling and a tapered die using three different compressive forces. The composition and axial and radial tensile strengths of the tablets are shown in Table 6. The tablets were found to be nonirritating to the esophagus.

Table 6

| Component | mg/tablet | | |
|---|---|---|---|
| Salicylsalicylic acid | 1000.0 | | |
| Avicel™ PH101 | 133.0 | | |
| Ac-Di-Sol™ | 8.0 | | |
| Hydroxypropyl cellulose LF | 48.0 | | |
| Magnesium stearate (dense) | 2.0 | | |
| Compressive force (kg) | 1090 | 1500 | 1910 |
| Axial TS (kg/cm$^2$) | 3.5±2.1 | 3.2±2.6 | 5.5±3.4 |
| Radial TS (kg/cm$^2$) | 8.8±1.0 | 11.6±1.1 | 14.6±1.0 |
| Axial/radial ratio | 0.40 | 0.28 | 0.38 |

Example 9

5,000 g of salicylsalicylic acid powder, 665 g of Avicel™ PH101, 40 g of Ac-Di-Sol™ and 240 g of hydroxypropyl cellulose LF were placed in a Fuji vertical granulator and dry mixed for 5 minutes with the blade speed set at 300 rpm and the cross-screw at low speed. The blade speed was set to 450 rpm and the cross-screw to high speed then water was sprayed under $10^5$ Pa (1 bar) of pressure and delivered at a rate of about 200 ml per minute. After 8 minutes the mass still looked dry and not uniformly wetted. It was beaten for 2 minutes without spraying. The blade speed was reduced to 200 rpm and water sprayed for another minute. A total of 1600 ml of water was sprayed. The mass was beaten for 1 minute then discharged. The granulation was dried in a fluid bed drier at 60 °C until the loss-on-drying was less than 1 % than oscillated through a 1.68 mm sieve opening (a 12 mesh screen). The bulk density was 0.593g/cc. 2,378g of the granulation was placed in a 18.4 l Hobart mixer and blended with 4.0 g of magnesium stearate (dense) for 4 minutes at low speed (#1). The granulation was compressed on a Stokes BB-2 press equipped with 12.7 mm flat faced beveled edged tooling and a tapered die using three different compressive forces. The composition of the tablets and the axial and radial tensile strengths are shown in Table 7. The tablets did not irritate the esophagus.

Table 7

| Component | mg/tablet | | |
|---|---|---|---|
| Salicylsalicylic acid | 1000.0 | | |
| Avicel™ PH101 | 133.0 | | |
| Ac-Di-Sol™ | 8.0 | | |
| Hydroxypropyl cellulose LF | 48.0 | | |
| Magnesium stearate (dense) | 2.0 | | |
| Compressive force (kg) | 1090 | 1500 | 1910 |
| Axial TS (kg/cm$^2$) | $2.9\pm2.0$ | $3.6\pm2.8$ | $3.8\pm2.7$ |
| Radial TS (kg/cm$^2$) | $8.6\pm0.7$ | $11.5\pm0.9$ | $14.3\pm0.9$ |
| Axial/radial ratio | 0.34 | 0.31 | 0.27 |

Example 10

A 18.4 l bowl of a Hobart mixer was charged with 3,750 g of salicylsalicylic acid powder, 500.0 g of Avicel™ PH101 and 30.0 g of Ac-Di-Sol™. The powders were blended for 10 minutes at speed #1. 1,487 g of a 12.1% solution of hydroxypropyl cellulose LF was added while mixing at speed #1. The resulting mixture was granulated for 3.5 minutes. The granulation was dried in a fluid bed drier at 60°C for 1 hour then oscillated through a 2.38 mm sieve opening (a 0.039 inch screen). 4173g of the granulation was placed in a 18.4 L bowl on a Hobart mixer. Approximately one fourth of the granulation was removed, placed in a plastic bag and mixed with 18.7 g of magnesium stearate (dense). This premix was added to the the remainder of the granulation in the Hobart bowl and blended for 1 minute at speed #1. A portion of the granulation was compressed on a Stokes BB-2 press equipped with flat faced beveled edged tooling and a tapered die at three different compressive forces. These tablets were used for the tensile strength measurements (Table 8). The remainder of the granulation was compressed on a Stokes BB-2 press equipped with 7.9 mm by 19.0 mm standard cup upper bisect tooling and a tapered die. These tablets were used in the determination of hardness, friability, disintegration, and dissolution. The tablets were found to be nonirritating to the esophagus. The composition and characteristics of the tablets are shown in Table 8.

## Table 8

| Component | mg/tablet | | |
|---|---|---|---|
| Salicylsalicylic acid | 750.0 | | |
| Avicel™ PH101 | 100.0 | | |
| Ac-Di-Sol™ | 6.0 | | |
| Hydroxypropyl cellulose LF | 36. | | |
| Magnesium stearate (dense) | 4.0 | | |
| Tablet Characteristics | | | |
| Hardness (kp) | 20 | | |
| Friability | 0.0 % | | |
| Disintegration time (minutes) | $4.97 \pm 1.08$ | | |
| Dissolution (n=6) | $96.2 \pm 2.7$ % | | |
| Compressive force (kg) | 1090 | 1500 | 1910 |
| Axial TS (kg/cm$^2$) | $6.3 \pm 4.1$ | $6.6 \pm 3.4$ | $6.8 \pm 3.8$ |
| Radial TS (kg/cm$^2$) | $13.6 \pm 1.3$ | $17.7 \pm 1.6$ | $20.4 \pm 1.1$ |
| Axial/radial ratio | 0.46 | 0.37 | 0.33 |

Examples 11-18

Using the general method of example 10, tablets with the composition and characteristics shown in Tables 9 and 10 were prepared. The tablets were formed on a Stokes BB-2 press equipped with 7.9 mm by 19.0 mm standard cup upper bisect tooling and a tapered die using three different compressive forces.

## Table 9

### mg/tablet

| Example | SSA | Avicel™ | HPC | Ac-Di-Sol™ | MgSt |
|---------|-----|---------|-----|------------|------|
| 11 | 750.0 | 100.0 | 30.0 | 4.0 | 4.0 |
| 12 | 750.0 | 100.0 | 42.0 | 4.0 | 4.0 |
| 13 | 750.0 | 100.0 | 30.0 | 8.0 | 4.0 |
| 14 | 750.0 | 100.0 | 42.0 | 8.0 | 4.0 |
| 15 | 750.0 | 100.0 | 36.0 | 2.0 | 4.0 |
| 16 | 750.0 | 100.0 | 36.0 | 10.0 | 4.0 |
| 17 | 750.0 | 100.0 | 26.0 | 6.0 | 4.0 |
| 18 | 750.0 | 100.0 | 46.0 | 6.0 | 4.0 |

SSA = salicylsalicylic acid; HPC = hydroxypropyl cellulose

MgSt = magnesium stearate

## Table 10

Compressive Force

| (kg) | 1090 | 1500 | 1910 | 1090 | 1500 | 1910 |
|------|------|------|------|------|------|------|
| Example | | Hardness (kp) | | | Dissolution (%) | |
| 11 | 15.6 | 18.1 | 20.5 | $105.0^6$ | * | $103.8^5$ |
| 12 | 15.6 | 18.5 | 20.1 | $100.1^6$ | * | $108.5^6$ |
| 13 | 14.6 | 16.3 | 18.9 | $102.7^6$ | * | $107.9^5$ |
| 14 | 14.0 | 17.3 | 21.4 | $97.7^6$ | * | $100.6^6$ |
| 15 | 14.8 | 17.1 | * | * | $98.1^3$ | $104.1^5$ |
| 16 | 14.6 | 16.6 | 19.8 | * | $100^3$ | * |
| 17 | 13.7 | 16.5 | 19.6 | * | $100^2$ | * |
| 18 | 15.7 | 16.4 | 21.0 | * | $92.3^5$ | * |

*Value not determined; the superscript indicates the number of tablets used to determine the value given.

## Claims

1. A tablet comprising:
   a) salsalate present in an amount of 80 to 90 percent by weight based on the total weight of the tablet;
   b) microcrystalline cellulose present in an amount of 5 to 15 percent by weight based on the total weight of the tablet;
   c) hydroxypropyl cellulose as a binder, substantially uniformly dispersed in the tablet and present in an amount of 1.5 to 5.5 percent by weight based on the total weight of the tablet;
   d) a lubricant present in an amount of 0.15 to 0.5 percent by weight based on the total weight of the tablet; and
   e) a superdisintegrant selected from crosslinked polyvinylpyrrolidone, sodium starch glycolate and crosslinked carboxymethyl cellulose present in an amount of 0.2 to 1.5 percent by weight based on the total weight of the tablet,
      which tablet exhibits at least 85% dissolution when tested according to the Dissolution Assay.

2. A tablet according to Claim 1 wherein the superdisintegrant is croscarmellose sodium (Type A).

3. A tablet according to any preceding claim wherein the hydroxypropyl cellulose has a molecular weight from 75,000 to 125,000.

4. A tablet according to any preceding claim wherein the lubricant is magnesium stearate.

5. A tablet comprising:
   a) salsalate present in an amount of 81 to 89 percent by weight based on the total weight of the tablet;
   b) microcrystalline cellulose present in an amount of 5 to 12 percent by weight based on the total weight of the tablet;
   c) hydroxypropyl cellulose as a binder, substantially uniformly dispersed in the tablet and present in an amount of 1.1 to 2.5 percent by weight based on the total weight of the tablet;
   d) a lubricant present in an amount of 0.15 to 0.4 percent by weight based on the total weight of the tablet; and
   e) corn starch present in an amount of 3.5 to 8 percent by weight based on the total weight of the tablet,
      which tablet exhibits at least 85% dissolution when tested according to the Dissolution Assay.

6. A tablet as claimed in claim 5 comprising:
   a) salsalate present in an amount of 84.4 percent by weight based on the total weight of the tablet;
   b) microcrystalline cellulose present in an amount of 5.6 percent by weight based on the total weight of the tablet;
   c) hydroxypropyl cellulose LF present in an amount of 2 percent by weight based on the total weight of the tablet;
   d) magnesium stearate present in an amount of 0.2 percent based on the total weight of the tablet; and
   e) corn starch present in an amount of 7.8 percent by weight based on the total weight of the tablet.

7. A tablet comprising:
   a) salsalate present in an amount of 81 to 85 percent by weight based on the total weight of the tablet;
   b) microcrystalline cellulose present in an amount of 13 to 18 percent by weight based on the total weight of the tablet;
   c) hydroxypropyl cellulose as a binder, substantially uniformly dispersed in the tablet and present in an amount of 1 to 5 percent by weight based on the total weight of the tablet; and
   d) a lubricant present in an amount of 0.1 to 0.5 percent by weight based on the total weight of the tablet,
      which tablet exhibits at least 85% dissolution when tested according to the Dissolution Assay.

## Patentansprüche

1. Tablette umfassend:
   a) Salsalat in einer Menge von 80 bis 90 Gew.-% basierend auf dem Gesamtgewicht der Tablette;
   b) mikrokristalline Cellulose in einer Menge von 5 bis 15 Gew.-% basierend auf dem Gesamtgewicht der Tablette;
   c) Hydroxypropylcellulose als Bindemittel, das im wesentlichen gleichmäßig in der Tablette verteilt und in einer Menge von 1,5 bis 5,5 Gew.-% basierend auf dem Gesamtgewicht der Tablette vorhanden ist;

d) ein Gleitmittel in einer Menge von 0,15 bis 0,5 Gew.-% basierend auf dem Gesamtgewicht der Tablette; und

e) ein hochwirksames Abbaumittel, das ausgewählt ist aus der Gruppe umfassend vernetztes Polyvinylpyrrolidon, Natriumstärkeglycolat und vernetzte Carboxymethylcellulose in einer Menge von 0,2 bis 1,5 Gew.-% basierend auf dem Gesamtgewicht der Tablette,

wobei die Tablette sich mindestens zu 85% löst, wenn sie in einem Lösungstest getestet wird.

2. Tablette nach Anspruch 1, dadurch gekennzeichnet, daß das hochwirksame Abbaumittel Croscarmellose-Natrium (Typ A) ist.

3. Tablette nach einem der vorhergehenden Ansprüche, dadurch gekennzeichnet, daß die Hydroxypropylcelllulose eine relative Molekülmasse von 75.000 bis 125.000 besitzt.

4. Tablette nach einem der vorhergehenden Ansprüche, dadurch gekennzeichnet, daß das Gleitmittel Magnesiumstearat ist.

5. Tablette umfassend:

a) Salsalat in einer Menge von 81 bis 89 Gew.-% basierend auf dem Gesamtgewicht der Tablette;

b) mikrokristalline Zellulose in einer Menge von 5 bis 12 Gew.-% basierend auf dem Gesamtgewicht der Tablette;

c) Hydroxypropylcellulose als Bindemittel, das im wesentlichen gleichmäßig in der Tablette verteilt und in einer Menge von 1,1 bis 2,5 Gew.-% basierend auf dem Gesamtgewicht der Tablette vorhanden ist;

d) ein Gleitmittel in einer Menge von 0,15 bis 0,4 Gew.-% basierend auf dem Gesamtgewicht der Tablette; und

e) Maisstärke in einer Menge von 3,5 bis 8 Gew.-% basierend auf dem Gesamtgewicht der Tablette,

wobei die Tablette sich mindestens zu 85% löst, wenn sie in einem Lösungstest getestet wird.

6. Tablette nach Anspruch 5, umfassend:

a) Salsalat in einer Menge von 84.4 Gew.-% basierend auf dem Gesamtgewicht der Tablette;

b) mikrokristalline Cellulose in einer Menge von 5,6 Gew.-% basierend auf dem Gesamtgewicht der Tablette;

c) Hydroxypropylcellulose LF in einer Menge von 2 Gew.-% basierend auf dem Gesamtgewicht der Tablette;

d) Magnesiumstearat in einer Menge von 0,2 Gew.-% basierend auf dem Gesamtgewicht der Tablette; und

e) Maisstärke in einer Menge von 7,8 Gew.-% basierend auf dem Gesamtgewicht der Tablette.

7. Tablette umfassend:

a) Salsalat in einer Menge von 81 bis 85 Gew.-% basierend auf dem Gesamtgewicht der Tablette;

b) mikrokristalline Cellulose in einer Menge von 13 bis 18 Gew.-% basierend auf dem Gesamtgewicht der Tablette;

c) Hydroxypropylcellulose als Bindemittel, das im wesentlichen gleichmäßig in der Tablette verteilt und in einer Menge von 1 bis 5 Gew.-% basierend auf dem Gesamtgewicht der Tablette vorhanden ist; und

d) ein Gleitmittel in einer Menge von 0,1 bis 0,5 Gew.-% basierend auf dem Gesamtgewicht der Tablette,

wobei die Tablette sich mindestens zu 85% löst, wenn sie in einem Lösungstest getestet wird.

**Revendications**

1. Pastille comprenant :

a) du salsalate présent en quantité de 80 à 90% en poids rapportée au poids total de la pastille;

b) de la cellulose microcristalline présente en quantité de 5 à 15% en poids rapportée au poids total de la pastille;

c) de l'hydroxypropylcellulose comme liant dispersée de manière essentiellement uniforme dans la pastille et présente en quantité de 1,5 à 5,5% en poids rapportée au poids total de la pastille;

d) un lubrifiant présent en quantité de 0,15 à 0,5% en poids rapportée au poids total de la partie; et

e) un super-désintégrant choisi parmi le polyvinylpyrrolidone réticulé, sel sodique du glycolate d'amidon et la carboxyméthylcellulose réticulée présents en quantité de 0,2 à 1,5% en poids rapportée au poids total de la pastille,

ladite pastille présentant une dissolution d'au moins 85% lorsqu'on la teste suivant l'essai de dissolution.

2. Pastille selon la revendication 1, dans laquelle le super-désintégrant est le sel sodique du croscarmellose (type A).

3. Pastille selon l'une quelconque des revendications précédentes, dans laquelle l'hydroxypropylcellulose à un poids moléculaire de 75.000 à 125.000.

4. Pastille selon l'une quelconque des revendications précédentes, dans laquelle le lubrifiant est du stéarate de magnésium.

5. Pastille comprenant :
a) du salsalate présent en quantité de 81 à 89% en poids rapportée au poids total de la pastille;
b) de la cellulose microcristalline présente en quantité de 5 à 12% en poids rapportée au poids total de la pastille;
c) de l'hydroxypropylcellulose comme liant dispersée de manière essentiellement uniforme dans la pastille et présente en quantité de 1,1 à 2,5% en poids rapportée au poids total de la pastille;
d) un lubrifiant présent en quantité de 0,15 à 0,4% en poids rapportée au poids total de la pastille; et
e) de l'amidon de maïs présent en quantité de 3,5 à 8% en poids rapportée au poids total de la pastille,
ladite pastille présentant une dissolution d'au moins 85% lorsqu'on la teste suivant l'essai de dissolution.

6. Pastille selon la revendication 5, comprenant :
a) du salsalate présent en quantité de 84,4% en poids rapportée au poids total de la pastille;
b) de la cellulose microcristalline présente en quantité de 5,6% en poids rapportée au poids total de la pastille;
c) de l'hydroxypropylcellulose LF présente en quantité égale à 2% en poids rapportée au poids total de la pastille;
d) du stéarate de magnésium présent en quantité de 0,2% rapportée au poids total de la pastille; et
e) de l'amidon de maïs présent en quantité de 7,8 % en poids rapportée au poids total de la pastille.

7. Pastille comprenant :
a) du salsalate présent en quantité de 81 à 85% en poids rapportée au poids total de la pastille;
b) de la cellulose microcristalline présente en quantité de 13 à 18% en poids rapportée au poids total de la pastille;
c) de l'hydroxypropylcellulose servant de liant, dispersée de manière essentiellement uniforme dans la pastille et présente en quantité de à 1 à 5% en poids rapportée au poids total de la pastille; et
d) un lubrifiant présent en quantité de 0,1 à 0,5% en poids rapportée au poids total de la pastille,
ladite pastille présentant une dissolution d'au moins 85% lorsqu'on la teste suivant l'essai de dissolution.